# EUROPEAN PATENT SPECIFICATION

(11) **EP 4 529 229 B1**
(45) Date of publication and mention of the grant of the patent: **25.03.2026**
(21) Application number: 24202113.7
(22) Date of filing: 24.09.2024
(51) Int. Cl.: H04R 25/00

(54) **A HEARING AID COMPRISING A WIRELESS AUDIO RECEIVER AND AN OWN-VOICE DETECTOR**
HÖRGERÄT MIT EINEM DRAHTLOSEN AUDIOEMPFÄNGER UND EINEM DETEKTOR ZUR DETEKTION DER EIGENEN STIMME
PROTHÈSE AUDITIVE COMPRENANT UN RÉCEPTEUR AUDIO SANS FIL ET UN DÉTECTEUR DE SA PROPRE VOIX

(30) Priority: 25.09.2023 EP 23199298
(43) Date of publication of application: 26.03.2025
(60) Divisional application: 26157871.0
(73) Proprietor: Oticon A/S, 2765 Smørum (DK)
(72) Inventor: TREUE, Franz, DK-2765 Smørum (DK)
(74) Representative: Demant

(56) References cited:
- US-A1- 2011 137 649
- US-A1- 2019 261 097

## Description

### TECHNICAL FIELD

The present application relates to the field of hearing aids, in particular to own-voice detection, or the use thereof in a hearing aid.

US2011137649A1 deals with a hearing aid configured to control the gain applied to a microphone input signal and to an audio stream input in dependence of a control signal indicative of the presence of the voice of the user ('own voice') of the hearing aid.

### SUMMARY

Receiving direct audio (from an audio delivery device, e.g. from a telephone, a remote microphone, or a television) is a standard feature in state of the art hearing aids. The present disclosure is related to the use of own-voice detection to control the mixing ratio between sound from the environment (picked up by a microphone) and a received (direct, e.g. streamed) audio input before a resulting signal is presented to the user of the hearing aid via an output transducer.

In a typical hearing aid, there is a fixed scheme for setting the gain of a microphone path, when audio is received, e.g. to attenuate the microphone input by 20 dB.

When the audio input is related to a telephone conversation, it is likely that the user really wants to attenuate all sound from the surrounding, e.g. even introduce noise cancelation of external "noise" (e.g. in open fittings).

But when the audio input is related to an audio stream, e.g. comprising TV sound, or sound picked up by a remote microphone (e.g. a partner or 'teacher' microphone), the choice is not that obvious. In a TV sound setup, where the hearing aid user is not alone in the room, it is likely that a conversation (e.g. regarding what to watch or what is being watched) may be desired. But it could also be that focus on the TV sound is preferred.

In an education setting (e.g. using a remote microphone) similar issues may prevail. While the student (e.g. a hearing aid user) is listening to a teacher, a fellow student next to the student may (or may not!) be of interest to listen to (via the microphone input).

Several other use-case like this can be thought of but a strategy for (automatically) controlling the gain applied to a hearing aid microphone input when a direct audio input is received is needed.

The idea is to use own-voice detection to decide on the microphone attenuation in dependence of the source of the audio input.

When a phone-call is received, an attenuation of the environment sound is likely desired, e.g. an attenuation of a fixed amount, e.g. 20 dB, or more, or an attenuation in dependence of a current input level (e.g. a larger attenuation the larger the current input level is, e.g. at the start of the direct audio reception, or adaptively adjusted to the input level over time during the audio reception).

When in a TV streaming situation, the own-voice detection can be used to disable the attenuation. If you are watching TV with a partner, a certain default attenuation of the microphone input is desired. But only until you start a parallel conversation with the partner. The microphone input should not be completely turned off, so that a conversation can start. In other words, the default attenuation should be set to a value that would allow a normal-level input to be audible to the hearing aid user. When a conversation with the partner is started, attenuation of the microphone input should be disabled. The own-voice detector can be used to detect the start of the conversation. If the hearing aid user speaks it is more than likely that a reply from one or more persons in the room can be expected.

Hence, when the user is in a streaming situation receiving audio from a wireless audio transmission (e.g. Bluetooth, etc.) device AND when this is NOT a phone conversation, own-voice detection may preferably be used to temporarily remove microphone attenuation (at least for a period of time, e.g. between 2 and 10 seconds).

Preferably, the scheme according to the present disclosure for controlling gain of sound picked up by the hearing aid microphone(s) when direct audio is received is automatically applied.

The hearing aid may be configured to individually enable or disable (select on/off) the microphone attenuation for
- reception of TV sound (e.g. enable),
- reception of sound from a remote communication partner (telephone mode) (e.g. disable),
- reception of sound from remote a microphone in the environment, etc. (e.g. enable).

### A hearing aid:

In an aspect of the present application, a hearing aid configured to be worn by a user, and as claimed in claim 1, is provided. The hearing aid comprises:
- a microphone configured to provide an electric input signal representative of sound from an environment around the user;
- a wireless receiver unit configured to receive a wireless signal from a transmitter of another device or system and to provide an audio input signal based thereon, and to identify said audio input signal as originating from one of a multitude of different types of audio transmitters;
- an own voice detector configured to provide an own voice control signal indicative of whether or not or with what probability the user's own voice is present in said sound from the environment of the user;
- a mixer configured to provide a mixed signal comprising a mixture of said electric input signal, or a signal originating therefrom and said audio input signal, or a signal originating therefrom;
- an input gain controller configured to apply an input gain to said electric input signal, or to a signal originating therefrom, at least when said hearing aid is in a wireless reception mode, wherein said wireless receiver unit receives a signal from at least one of said multitude of different types of audio transmitters; and
- an output transducer for providing stimuli representative of said mixed signal or a signal originating therefrom, perceivable as sound to the user.

The input gain controller is configured to apply an input gain to the electric input signal, or to a signal originating therefrom in dependence of a) the own voice control signal and b) the type of audio transmitter.

Thereby an improved hearing aid may be provided.

The wireless receiver unit may be configured to provide an audio transmitter type control signal indicative of the origin of a currently received wireless signal. The input gain controller may be configured to apply the input gain to the electric input signal, or to a signal originating therefrom, in dependence of the audio transmitter type control signal.

The input gain controller may be configured to determine the type of audio transmitter from a current mode of operation of the hearing aid. The hearing aid may be configured to operate in a multitude of different modes, e.g. a normal mode, and one or more specific modes, e.g. selectable by a user via a user interface, or automatically selectable. A mode of operation may be optimized to a specific acoustic situation or environment, e.g. a communication (or 2-way audio) mode, such as a telephone mode (where the hearing aid is configured to receive audio from an audio transmitter of a telephone device and to transmit audio (the hearing aid user's voice) to a telephone device), or a partner microphone mode (wherein the hearing aid is configured to receive audio from an audio transmitter of a portable microphone), or a table microphone mode (where the hearing aid is configured to receive audio from an audio transmitter of a stationary (e.g. table-) microphone unit), TV-reception mode (where the hearing aid is configured to receive audio from an audio transmitter of a TV), etc.

The current mode of operation of the hearing aid may be determined by the user via a user interface. The hearing aid may comprise a user interface allowing the user to control functionality of the hearing aid, including to enter (and/or leave) a specific one of a multitude of modes of operation (e.g. represented by different hearing aid programs).

At least two of the multitude of different types of audio transmitters may use different audio transmission formats.

The multitude of different types of audio transmitters may comprise one or more of a video-sound-transmitter, a table microphone transmitter, a portable microphone transmitter, and a telephone transmitter.

Audio transmission formats may comprise standardized or proprietary audio transmission formats. For example, an audio transmission format of the different audio transmission formats comprises a standardized or proprietary audio transmission format. For example, each of the different audio transmission formats comprises a standardized or proprietary audio transmission format. The different audio transmission formats may be construed as a plurality (e.g., a multitude) of audio transmission formats.

The audio transmission formats may e.g. be used in one or more of Bluetooth, Bluetooth Low Energy, and LE audio protocols.

The hearing aid may comprise an other-voice detector configured to provide an other-voice-control signal indicative of whether or not or with what probability another voice than the user's own voice is present in the sound from the environment of the user. The hearing aid may comprise a general voice activity detector. The other-voice detector may comprise a combination of the own voice detector and a general voice activity detector. The other voice detector may be configured to identify the voice of one or more specific other persons, e.g. voices of persons typically present in the acoustic environments related to streaming. Such persons may be a spouse or children or other persons frequently engaging with the hearing aid user. Thereby the particular gain modification according to the present disclosure may be limited to situations involving such one or more specific other persons (e.g. to conversations involving specific other persons, e.g. when the hearing aid is in a TV-reception mode).

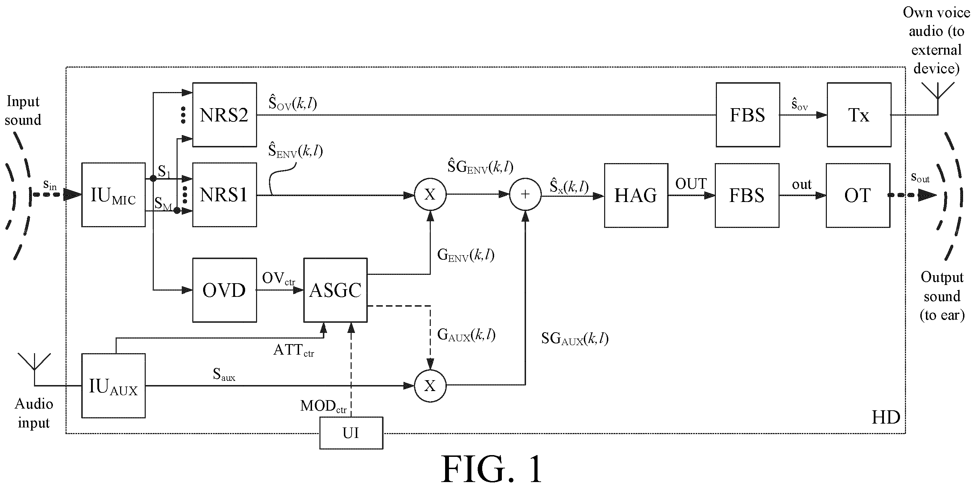

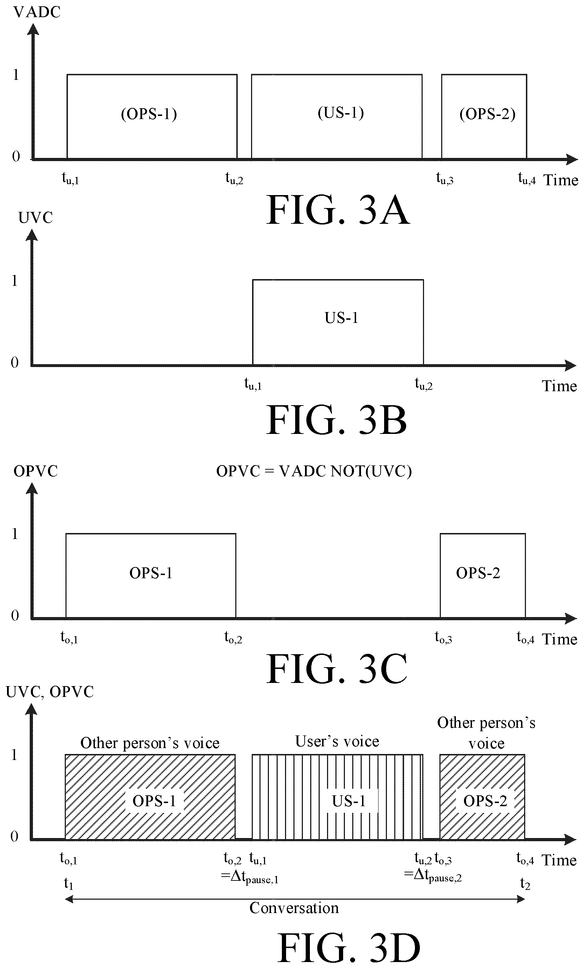

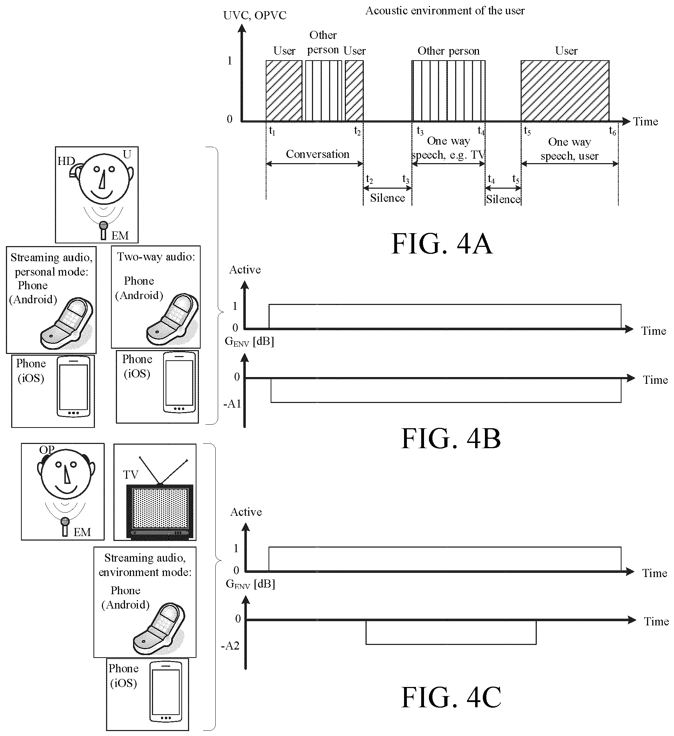

The hearing aid may comprise a conversation detector identifying a conversation that the user is currently engaged in, and to provide a conversation control signal indicative thereof. The conversation detector may be based on the own voice control signal in combination with a timer. When the own voice detection signal (OV_{ctr}) indicates that the user's voice is present (e.g. OV_{ctr}='active' in FIG. 1), a conversation may be assumed, and the conversation control signal set to 'active' (indicative of a conversation involving the user having been detected, cf. e.g. FIG. 4A). When the user 's voice is no longer detected, the conversation control signal is kept 'active' for a predefined or adaptively determined time period (Δtₚₐᵤₛₑ). If the own voice detection signal (or a general voice activity control signal) is activated again within the time period (cf. e.g. Δt_{pause,n}, n= 1, 2 in FIG. 3D), the conversation control signal is kept 'active', and so on). If the own voice detection signal (or a general voice activity control signal) is NOT 'active' again (i.e. no own voice detected) within the time period (Δtₚₐᵤₛₑ), the conversation control signal is deactivated (thereby indicating that a conversation is no longer assumed), see e.g. FIG. 4A. During the 'conversation' (where the conversation control signal is 'active'), the input gain applied to the electric input signal from the input transducer(s), e.g. microphone(s), or to a signal originating therefrom (S_{ENV}, in FIG. 1) is increased, with a predetermined or dynamically determined amount (e.g. by 3-12 dB). The time period (Δtₚₐᵤₛₑ) may preferably be adapted to a normal maximum time between 'turns' of a conversation, e.g. empirically determined. The time period (Δtₚₐᵤₛₑ) may e.g. be set to in the range from 2 to 10 s, e.g. from 2 to 5 s.

The conversation detector may e.g. be based on the own voice control signal and the other-voice-control signal. The conversation detector may further be based on an analysis of the mutual timing of the own voice control signal, the other-voice-control signal and predefined rules regarding time-overlap and/or time gaps between the two control signals.

The input gain controller may be configured to apply the input gain to the electric input signal, or to a signal originating therefrom in dependence of a) the own voice control signal, b) the type of audio transmitter, and c) the conversation control signal.

The input gain controller may be configured to apply an input gain to the audio input signal. The input gain applied to said audio input signal may e.g. be dependent on a current input level as observed by the microphone of the hearing aid. The input gain applied to said audio input signal may e.g. be dependent on the current mode of operation, e.g. a 2-way audio mode, such as a 'telephone mode' of operation.

The input gain controller may be configured to apply an input gain to the electric input signal and/or to the audio input signal to provide a certain mixing ratio of the mixed signal.

The hearing aid may comprise one or more electrical sensors configured to be located close to the ear and close to skin of the user when the hearing aid is worn by the user.

The hearing aid may be configured to extract electroencephalography (EEG) and/or electromyography (EMG) signals from the one of more electrical sensors.

The own voice detector may be based on an analysis of the EEG and/or EMG signals.

The own voice control signal may be determined from a high pass filtered part of the EEG and/or EMG signal(s). The own voice control signal may be determined from EEG and/or EMG signal(s) evoked by muscle activity. Speech or preparation of speech is characterized by high-frequency, high-amplitude (EEG-and/or EMG-) signals.

The hearing aid may be adapted to provide a frequency dependent gain and/or a level dependent compression and/or a transposition (with or without frequency compression) of one or more frequency ranges to one or more other frequency ranges, e.g. to compensate for a hearing impairment of a user. The hearing aid may comprise a signal processor for enhancing the input signals and providing a processed output signal.

The hearing aid may comprise an output unit for providing a stimulus perceived by the user as an acoustic signal based on a processed electric signal. The output unit may comprise a number of electrodes of a cochlear implant (for a CI type hearing aid) or a vibrator of a bone conducting hearing aid. The output unit may comprise an output transducer. The output transducer may comprise a receiver (loudspeaker) for providing the stimulus as an acoustic signal to the user (e.g. in an acoustic (air conduction based) hearing aid). The output transducer may comprise a vibrator for providing the stimulus as mechanical vibration of a skull bone to the user (e.g. in a bone-attached or bone-anchored hearing aid). The output unit may (additionally or alternatively) comprise a transmitter for transmitting sound picked up-by the hearing aid to another device, e.g. a far-end communication partner (e.g. via a network, e.g. in a telephone mode of operation, or in a headset configuration).

The hearing aid may comprise an input unit for providing an electric input signal representing sound. The input unit may comprise an (acousto-electric) input transducer, e.g. a microphone, for converting an input sound to an electric input signal. The input unit may comprise a wireless receiver for receiving a wireless signal comprising or representing sound and for providing an electric input signal representing said sound.

The wireless receiver and/or transmitter may e.g. be configured to receive and/or transmit an electromagnetic signal in the radio frequency range (3 kHz to 300 GHz). The wireless receiver and/or transmitter may e.g. be configured to receive and/or transmit an electromagnetic signal in a frequency range of light (e.g. infrared light 300 GHz to 430 THz, or visible light, e.g. 430 THz to 770 THz).

The hearing aid may comprise a directional microphone system adapted to spatially filter sounds from the environment, and thereby enhance a target acoustic source among a multitude of acoustic sources in the local environment of the user wearing the hearing aid. The directional system may be adapted to detect (such as adaptively detect) from which direction a particular part of the microphone signal originates. This can be achieved in various different ways as e.g. described in the prior art. In hearing aids, a microphone array beamformer is often used for spatially attenuating background noise sources. The beamformer may comprise a linear constraint minimum variance (LCMV) beamformer. Many beamformer variants can be found in literature. The minimum variance distortionless response (MVDR) beamformer is widely used in microphone array signal processing. Ideally the MVDR beamformer keeps the signals from the target direction (also referred to as the look direction) unchanged, while attenuating sound signals from other directions maximally. The generalized sidelobe canceller (GSC) structure is an equivalent representation of the MVDR beamformer offering computational and numerical advantages over a direct implementation in its original form.

The hearing aid may comprise antenna and transceiver circuitry allowing a wireless link to an entertainment device (e.g. a TV-set), a communication device (e.g. a telephone), a wireless microphone, or another hearing aid, etc. The hearing aid may thus be configured to wirelessly receive a direct electric input signal from another device. Likewise, the hearing aid may be configured to wirelessly transmit a direct electric output signal to another device. The direct electric input or output signal may represent or comprise an audio signal and/or a control signal and/or an information signal.

In general, a wireless link established by antenna and transceiver circuitry of the hearing aid can be of any type. The wireless link may be a link based on near-field communication, e.g. an inductive link based on an inductive coupling between antenna coils of transmitter and receiver parts. The wireless link may be based on far-field, electromagnetic radiation. Preferably, frequencies used to establish a communication link between the hearing aid and the other device is below 70 GHz, e.g. located in a range from 50 MHz to 70 GHz, e.g. above 300 MHz, e.g. in an ISM range above 300 MHz, e.g. in the 900 MHz range or in the 2.4 GHz range or in the 5.8 GHz range or in the 60 GHz range (ISM=Industrial, Scientific and Medical, such standardized ranges being e.g. defined by the International Telecommunication Union, ITU). The wireless link may be based on a standardized or proprietary technology. The wireless link may be based on Bluetooth technology (e.g. Bluetooth Low-Energy technology, e.g. LE audio), or Ultra WideBand (UWB) technology.

The hearing aid may be or form part of a portable (i.e. configured to be wearable) device, e.g. a device comprising a local energy source, e.g. a battery, e.g. a rechargeable battery. The hearing aid may e.g. be a low weight, easily wearable, device, e.g. having a total weight less than 100 g, such as less than 20 g, such as less than 5 g.

The hearing aid may comprise a 'forward' (or 'signal') path for processing an audio signal between an input and an output of the hearing aid. A signal processor may be located in the forward path. The signal processor may be adapted to provide a frequency dependent gain according to a user's particular needs (e.g. hearing impairment). The hearing aid may comprise an 'analysis' path comprising functional components for analyzing signals and/or controlling processing of the forward path. Some or all signal processing of the analysis path and/or the forward path may be conducted in the frequency domain, in which case the hearing aid comprises appropriate analysis and synthesis filter banks. Some or all signal processing of the analysis path and/or the forward path may be conducted in the time domain.

An analogue electric signal representing an acoustic signal may be converted to a digital audio signal in an analogue-to-digital (AD) conversion process, where the analogue signal is sampled with a predefined sampling frequency or rate fₛ, fₛ being e.g. in the range from 8 kHz to 48 kHz (adapted to the particular needs of the application) to provide digital samples xₙ (or x[n]) at discrete points in time tₙ (or n), each audio sample representing the value of the acoustic signal at tₙ by a predefined number N_{b} of bits, N_{b} being e.g. in the range from 1 to 48 bits, e.g. 24 bits. Each audio sample is hence quantized using N_{b} bits (resulting in 2^{Nb} different possible values of the audio sample). A digital sample x has a length in time of 1/fₛ, e.g. 50 µs, for *fₛ* = 20 kHz. A number of audio samples may be arranged in a time frame. A time frame may comprise 64 or 128 audio data samples. Other frame lengths may be used depending on the practical application.

The hearing aid may comprise an analogue-to-digital (AD) converter to digitize an analogue input (e.g. from an input transducer, such as a microphone) with a predefined sampling rate, e.g. 20 kHz. The hearing aids may comprise a digital-to-analogue (DA) converter to convert a digital signal to an analogue output signal, e.g. for being presented to a user via an output transducer.

The hearing aid, e.g. the input unit, and or the antenna and transceiver circuitry may comprise a transform unit for converting a time domain signal to a signal in the transform domain (e.g. frequency domain or Laplace domain, Z transform, wavelet transform, etc.). The transform unit may be constituted by or comprise a TF-conversion unit for providing a time-frequency representation of an input signal. The time-frequency representation may comprise an array or map of corresponding complex or real values of the signal in question in a particular time and frequency range. The TF conversion unit may comprise a filter bank for filtering a (time varying) input signal and providing a number of (time varying) output signals each comprising a distinct frequency range of the input signal. The TF conversion unit may comprise a Fourier transformation unit (e.g. a Discrete Fourier Transform (DFT) algorithm, or a Short Time Fourier Transform (STFT) algorithm, or similar) for converting a time variant input signal to a (time variant) signal in the (time-)frequency domain. The frequency range considered by the hearing aid from a minimum frequency fₘᵢₙ to a maximum frequency fₘₐₓ may comprise a part of the typical human audible frequency range from 20 Hz to 20 kHz, e.g. a part of the range from 20 Hz to 12 kHz. Typically, a sample rate fₛ is larger than or equal to twice the maximum frequency fₘₐₓ, fₛ ≥ 2fₘₐₓ. A signal of the forward and/or analysis path of the hearing aid may be split into a number *NI* of frequency bands (e.g. of uniform width), where *NI* is e.g. larger than 5, such as larger than 10, such as larger than 50, such as larger than 100, such as larger than 500, at least some of which are processed individually. The hearing aid may be adapted to process a signal of the forward and/or analysis path in a number *NP* of different frequency channels (*NP* ≤ *NI*). The frequency channels may be uniform or non-uniform in width (e.g. increasing in width with frequency), overlapping or non-overlapping.

The hearing aid may be configured to operate in different modes, e.g. a normal mode and one or more specific modes, e.g. selectable by a user, or automatically selectable. A mode of operation may be optimized to a specific acoustic situation or environment, e.g. a communication mode, such as a telephone mode. A mode of operation may include a low-power mode, where functionality of the hearing aid is reduced (e.g. to save power), e.g. to disable wireless communication, and/or to disable specific features of the hearing aid.

The hearing aid may comprise a number of detectors configured to provide status signals relating to a current physical environment of the hearing aid (e.g. the current acoustic environment), and/or to a current state of the user wearing the hearing aid, and/or to a current state or mode of operation of the hearing aid. Alternatively or additionally, one or more detectors may form part of an *external* device in communication (e.g. wirelessly) with the hearing aid. An external device may e.g. comprise another hearing aid, a remote control, and audio delivery device, a telephone (e.g. a smartphone), an external sensor, etc.

One or more of the number of detectors may operate on the full band signal (time domain). One or more of the number of detectors may operate on band split signals ((time-) frequency domain), e.g. in a limited number of frequency bands.

The number of detectors may comprise a level detector for estimating a current level of a signal of the forward path. The detector may be configured to decide whether the current level of a signal of the forward path is above or below a given (L-)threshold value. The level detector operates on the full band signal (time domain). The level detector operates on band split signals ((time-) frequency domain).

The hearing aid may comprise a voice activity detector (VAD) for estimating whether or not (or with what probability) an input signal comprises a voice signal (at a given point in time). A voice signal may in the present context be taken to include a speech signal from a human being. It may also include other forms of utterances generated by the human speech system (e.g. singing). The voice activity detector unit may be adapted to classify a current acoustic environment of the user as a VOICE or NO-VOICE environment. This has the advantage that time segments of the electric microphone signal comprising human utterances (e.g. speech) in the user's environment can be identified, and thus separated from time segments only (or mainly) comprising other sound sources (e.g. artificially generated noise). The voice activity detector may be adapted to detect as a VOICE also the user's own voice. Alternatively, the voice activity detector may be adapted to exclude a user's own voice from the detection of a VOICE.

The hearing aid may comprise an own voice detector for estimating whether or not (or with what probability) a given input sound (e.g. a voice, e.g. speech) originates from the voice of the user of the system. A microphone system of the hearing aid may be adapted to be able to differentiate between a user's own voice and another person's voice and possibly from NON-voice sounds.

The number of detectors may comprise a movement detector, e.g. an acceleration sensor. The movement detector may be configured to detect movement of the user's facial muscles and/or bones, e.g. due to speech or chewing (e.g. jaw movement) and to provide a detector signal indicative thereof.

The hearing aid may comprise a classification unit configured to classify the current situation based on input signals from (at least some of) the detectors, and possibly other inputs as well. In the present context 'a current situation' may be taken to be defined by one or more of
a) the physical environment (e.g. including the current electromagnetic environment, e.g. the occurrence of electromagnetic signals (e.g. comprising audio and/or control signals) intended or not intended for reception by the hearing aid, or other properties of the current environment than acoustic);
b) the current acoustic situation (input level, feedback, etc.), and
c) the current mode or state of the user (movement, temperature, cognitive load, etc.);
d) the current mode or state of the hearing aid (program selected, time elapsed since last user interaction, etc.) and/or of another device in communication with the hearing aid.

The classification unit may be based on or comprise a neural network, e.g. a recurrent neural network, e.g. a trained neural network.

The hearing aid may comprise an acoustic (and/or mechanical) feedback control (e.g. suppression) or echo-cancelling system. Adaptive feedback cancellation has the ability to track feedback path changes over time. It is typically based on a linear time invariant filter to estimate the feedback path, but its filter weights are updated over time. The filter update may be calculated using stochastic gradient algorithms, including some form of the Least Mean Square (LMS) or the Normalized LMS (NLMS) algorithms. They both have the property to minimize the error signal in the mean square sense with the NLMS additionally normalizing the filter update with respect to the squared Euclidean norm of some reference signal.

The hearing aid may further comprise other relevant functionality for the application in question, e.g. compression, noise reduction, etc.

The hearing aid may comprise a hearing instrument, e.g. a hearing instrument adapted for being located at the ear or fully or partially in the ear canal of a user. A hearing system may comprise a speakerphone (comprising a number of input transducers (e.g. a microphone array) and a number of output transducers, e.g. one or more loudspeakers, and one or more audio (and possibly video) transmitters e.g. for use in an audio conference situation), e.g. comprising a beamformer filtering unit, e.g. providing multiple beamforming capabilities.

### A hearing system:

In a further aspect, a hearing system comprising a hearing aid as described above, in the 'detailed description of embodiments', AND an auxiliary device is moreover provided.

The hearing system may be adapted to establish a communication link between the hearing aid and the auxiliary device to provide that information (e.g. control and status signals, possibly audio signals) can be exchanged or forwarded from one to the other.

The auxiliary device may be constituted by or comprise a remote control, a smartphone, or other portable or wearable electronic device, such as a smartwatch or the like.

The auxiliary device may be constituted by or comprise a remote control for controlling functionality and operation of the hearing aid(s). The function of a remote control may be implemented in a smartphone, the smartphone possibly running an APP allowing to control the functionality of the audio processing device via the smartphone (the hearing aid(s) comprising an appropriate wireless interface to the smartphone, e.g. based on Bluetooth or some other standardized or proprietary scheme).

The auxiliary device may be constituted by or comprise an audio gateway device adapted for receiving a multitude of audio signals (e.g. from an entertainment device, e.g. a TV or a music player, a telephone apparatus, e.g. a mobile telephone or a computer, e.g. a PC, a wireless microphone, etc.) and adapted for selecting and/or combining an appropriate one of the received audio signals (or combination of signals) for transmission to the hearing aid.

The auxiliary device may be constituted by or comprise another hearing aid. The hearing system may comprise two hearing aids adapted to implement a binaural hearing system, e.g. a binaural hearing aid system.

### An APP:

In a further aspect, a non-transitory application, termed an APP, is furthermore provided by the present disclosure. The APP comprises executable instructions configured to be executed on an auxiliary device to implement a user interface for a hearing aid or a hearing system described above in the 'detailed description of embodiments', and in the claims. The APP may be configured to run on cellular phone, e.g. a smartphone, or on another portable device allowing communication with said hearing aid or said hearing system.

A 'hearing system' refers to a system comprising one or two hearing aids, and a 'binaural hearing system' refers to a system comprising two hearing aids and being adapted to cooperatively provide audible signals to both of the user's ears. Hearing systems or binaural hearing systems may further comprise one or more 'auxiliary devices', which communicate with the hearing aid(s) and affect and/or benefit from the function of the hearing aid(s). Such auxiliary devices may include at least one of a remote control, a remote microphone, an audio gateway device, an entertainment device, e.g. a music player, a wireless communication device, e.g. a mobile phone (such as a smartphone) or a tablet or another device, e.g. comprising a graphical interface. Hearing aids, hearing systems or binaural hearing systems may e.g. be used for compensating for a hearing-impaired person's loss of hearing capability, augmenting or protecting a normal-hearing person's hearing capability and/or conveying electronic audio signals to a person. Hearing aids or hearing systems may e.g. form part of or interact with public-address systems, active ear protection systems, handsfree telephone systems, car audio systems, entertainment (e.g. TV, music playing or karaoke) systems, teleconferencing systems, classroom amplification systems, etc.

Embodiments of the disclosure may e.g. be useful in applications such as hearing devices adapted receive audio signals acoustically and electromagnetically, to process signals based thereon, and to present a resulting signal to a user of the hearing device.

The invention is defined in the appended set of claims.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 shows an embodiment of a hearing aid according to the present disclosure,
FIG. 2A shows a first TV-scenario comprising a hearing system according to the present disclosure; and
FIG. 2B shows a second TV-scenario comprising a hearing system according to the present disclosure,
FIG. 3A-3D shows voice detection signals for identifying a conversation between the user and another person, where
FIG. 3A shows an exemplary output control signal VADC of a (general) voice activity detector;
FIG. 3B shows an exemplary output control signal UVC of an own voice detector;
FIG. 3C shows an exemplary control signal OPVC derived from the control signals VADC and UVC of FIG. 3A and 3B respectively; and
FIG. 3D shows an exemplary conversation identifier based on the control signals of FIG. 3A, 3B and 3C,
FIG. 4A schematically shows a time sequence of voice detection control signals reflecting a varying acoustic environment of the user of the hearing aid, including sub-sequences reflecting a varying degree of speech-participation by the user; and
FIG. 4B schematically shows an exemplary microphone gain modification versus time for a hearing aid according to the present disclosure when receiving streamed audio from first types of audio transmitters; and
FIG. 4C schematically shows an exemplary microphone gain modification versus time for a hearing aid according to the present disclosure when receiving streamed audio from second types of audio transmitters, and

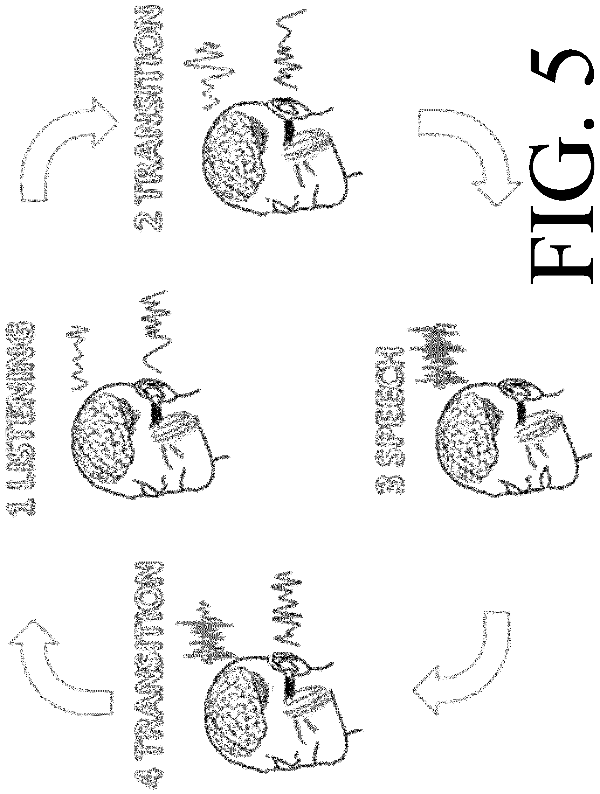

FIG. 5 schematically shows EEG signals originating from brain activity and muscle activity, respectively, of a hearing aid user in a listening situation (other voice) and a speech situation (own voice), respectively.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION OF EMBODIMENTS

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include micro-electronic-mechanical systems (MEMS), integrated circuits (e.g. application specific), microprocessors, microcontrollers, digital signal processors (DSPs), field programmable gate arrays (FPGAs), programmable logic devices (PLDs), gated logic, discrete hardware circuits, printed circuit boards (PCB) (e.g. flexible PCBs), and other suitable hardware configured to perform the various functionality described throughout this disclosure, e.g. sensors, e.g. for sensing and/or registering physical properties of the environment, the device, the user, etc. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

The present application relates to the field of hearing aids, in particular to a hearing aid configured to receive an audio input signal via a wireless receiver.

FIG. 1 shows an embodiment of a hearing aid according to the present disclosure. The hearing aid (HD) is configured to be worn by a user, e.g. at or in an ear of the user, e.g. fully or partially at or in an ear canal of the user. The hearing aid comprises an input unit (IU_{MIC}) comprising at least one input transducer (e.g. a microphone) configured to provide an electric input signal (S₁, ..., S_{M}, where M is larger than or equal to 1) representative of sound (cf. indication 'Input sound', sᵢₙ in the left part of FIG. 1) from an environment around the user. The hearing aid further comprises a wireless receiver unit (IU_{AUX}) comprising antenna and receiver circuitry configured to provide an audio input signal (Sₐᵤₓ) from another device or system. The hearing aid, e.g. the wireless receiver unit (IU_{AUX}), may e.g. further be configured to identify the origin of the audio input signal as a signal originating from one of a multitude of different types of transmitters, and to provide an audio transmitter type control signal (ATT_{ctr}) indicative thereof.

The hearing aid (HD) further comprises an own voice detector (OVD) configured to provide an own voce control signal (OV_{ctr}) indicative of whether or not, or with what probability the user's own voice is present in the sound (sᵢₙ) from the environment of the user. The hearing aid further comprises an input gain controller (ASGC) configured to apply an input gain (G_{ENV}, G_{AUX}) to the at least one electric input signal (S₁, ..., S_{M}) and/or to the audio input signal (Sₐᵤₓ), or to a signal or signals originating therefrom in dependence of the own voce control signal (OV_{ctr}) and/or in dependence of the audio transmitter type control signal (ATT_{ctr}).

The hearing aid may be configured to operate in different modes, e.g. a normal mode and one or more specific modes, e.g. a wireless reception mode, e.g. selectable by a user (e.g. via a user interface), or automatically selectable (cf. signal ATT_{ctr}). The type of audio transmitter that the hearing aid currently receives audio from may be defined by a specific mode of operation of the hearing aid (cf. e.g. mode control signal MOD_{ctr} from the user interface (UI)).

The receiver (Rx) or the input gain controller (ASGC) may be configured to (automatically) identify the type of audio transmitter that it currently is connected to (e.g. via a device identification parameter in the transmission protocol).

The input gain controller (ASGC) may be configured to (automatically) control (e.g. increase or decrease) the input gain (G_{ENV}) of the (here, noise reduced) microphone signal (Ŝ_{ENV}) in dependence of the own-voice detector (e.g. via the own voice detection control signal (OV_{ctr})), at least when the hearing aid is in a wireless reception mode, wherein the wireless receiver unit (IU_{AUX}) receives a signal from at least one of the multitude of different types of transmitters. In other words, the volume (as presented to the user) of the sound from the environment picked up by the at least one input transducer (or a noise reduced, e.g. beamformed version thereof) may be controlled in dependence of the own-voice detector and the type of transmitter (at least in the wireless reception mode).

In general, during time-periods where the user speaks, the volume (as presented to the user) of the sound picked up by the at least one input transducer (or a beamformed version thereof) may e.g. be attenuated compared to when the user does not speak (e.g. to ensure that the user's own voice (when played for the user) is not perceived as annoying by the user, i.e. to minimize the effect of occlusion). The scheme for controlling input gain(s) in dependence of own voice presence according to the present disclosure may be independent of such general approach.

In the exemplary embodiment of FIG. 1, the hearing aid (HD) comprises a first signal path (from input unit (IU_{MIC}) to output transducer (OT)) for applying a level and frequency dependent gain to an input signal of the hearing aid and to provide output stimuli representative thereof and perceivable as sound to the user (e.g. as acoustic sound to an ear of the user). The hearing aid further comprises a second signal path (from input unit (IU_{MIC}) to transmitter (Tx)) for providing an estimate of the user's own voice and transmitting it to an external device or system (e.g. to a telephone of the user). The hearing aid further comprises a third signal path (from a wireless receiver unit (IU_{AUX}) to a mixer ('+') located in the first signal path) for feeding an audio signal wirelessly received from another device or system to the user via the output transducer (OT) of the hearing aid. The input unit (IU_{MIC}) and the wireless receiver unit (IU_{AUX}) may comprise respective analysis filter banks to convert time domain input signals from the microphones and the wireless receiver, respectively to respective frequency sub-band signals (S₁, ..., S_{M}, S_{AUX}) in the time frequency domain (defined by respective frequency and time indices (*k,l*)).

The mixer is in the embodiment of FIG. 1 shown as an adder ('+') that adds the two streams together. In general, the mixer may be implemented as a mixer configured to provide a weighted mixture of a) the electric input signal, or a signal originating therefrom (Ŝ_{ENV}), and b) the audio input signal (S_{AUX}), or a signal originating therefrom. In the embodiment of FIG. 1, the noise reduced, e.g. beamformed electric input signal (Ŝ_{ENV}) is modified (weighted) by the input gain (G_{ENV}), and the audio input signal (S_{AUX}) is (optionally) modified (weighted) by the input gain (G_{AUX}). The sum of the weights may be equal to 1. The values of the weights may e.g. be implemented as α, 1-α, where 0 < α < 1, where e.g. α = G_{ENV}, and 1-α = G_{AUX}. Thereby the output of the mixer (Sx) is a weighted sum of the two 'input signals' (Ŝ_{ENV} and S_{AUX}), i.e. Sx = Ŝ_{ENV}G_{ENV} + S_{AUX}G_{AUX}. In certain modes of operation of the hearing aid, the input gain (G_{AUX}) applied to the audio input signal (S_{AUX}) is equal to 1.

In the embodiment of FIG. 1, each of the first and second signal paths comprises a noise reduction system (NRS1 and NRS2, respectively) for reducing noise in one or more signals picked up from the environment by microphones of the input unit (IN_{MIC}) and providing respective noise reduced signals (Ŝ_{ENV}, Ŝ_{OV}, respectively). The first noise reduction system (NRS1) may e.g. comprise a beamformer for reducing noise from one or more localized sound sources in the acoustic far-field environment of the hearing aid (e.g. ≥ 1 m away from the user) or to provide a substantially omni-directional output based on the inputs from a number of (individually omni-directional) microphones (and optionally to provide an estimate of localized (target) signal in the far-field environment of the hearing device). The first noise reduction system (NRS1) may e.g. provide its output signal as noise reduced signal Ŝ_{ENV}(*k*,*l*) in a time-frequency representation. The second noise reduction system (NRS1) may e.g. comprise an own voice beamformer configured to reduce noise from one or more localized sound sources in the environment of the hearing aid (and optionally to provide an estimate of the user's own voice). The second noise reduction system (NRS2) may e.g. provide its output signal as noise reduced signal Ŝ_{OV}(*k*,*l*) in a time-frequency representation (e.g. comprising an estimate of the user's own voice).

The first signal path is the main signal path (forward path) of the hearing device when 'only' implementing a classic hearing aid mode of operation.

The first signal path and a combination of the second and third signal paths are the main signal paths of the hearing device when a headset (or two-way audio) mode of operation is implemented. In case, no signals from the environment (picked up by the input unit (IU_{MIC})) are to be presented to the user, only the first and third signal paths are active in the headset mode.

The combination of the second and third signal paths are the main signal paths of the hearing device when a one-way-audio (or streaming audio) mode is implemented (cf. e.g. FIG. 4C). Again, in case, no signals from the environment (picked up by the input unit (IU_{MIC})) are to be presented to the user, only the third signal path is active in the one-way-audio mode.

The hearing aid (HD) comprises respective multiplication units ('X') configured to apply respective input gains (G_{ENV}, G_{AUX}) to the signals for the microphone path and direct audio input path, respectively. In the embodiment of FIG. 1, the gain modification is made in the forward path (first signal path) from the input unit (IU_{MIC}) to the output transducer (OT)), and/or in the third signal path from the wireless receiver unit (IU_{AUX}) to the output transducer (OT) of the first signal path (via the mixer ('+')).

A frequency and/or level dependent gain for compensating for a hearing impairment of the user (termed the 'hearing aid gain') may be provided by a 'hearing aid processor' (cf. block (HAG in FIG. 1) and applied after ('downstream' of) the input gain(s) (G_{ENV}, G_{AUX}) according to the present disclosure is(are) applied to the input signals (S₁, ..., S_{M}, Sₐᵤₓ) (or noise reduced versions thereof (Ŝ_{ENV}). The processed signal (OUT) provided by the 'hearing aid processor' (HAG) is converted to the time domain (cf. signal out) by a synthesis filter bank (FBS) and fed to the output transducer (OT) for presentation to the user as stimuli (sₒᵤₜ) perceivable as sound (cf. indication 'Output sound (to ear)' in the right part of FIG. 1). In case the processing of the forward path is in the time domain, the synthesis filter bank (FBS) can be dispensed with.

However, in a wireless reception mode, when a conversation is assumed to take place (see e.g. FIG. 3A-D, and FIG. 4, based in part on EP3930346A1 described below), however, another (or a further) strategy for controlling the volume of sound from the at least one input transducer (or a beamformed version thereof) may be applied.

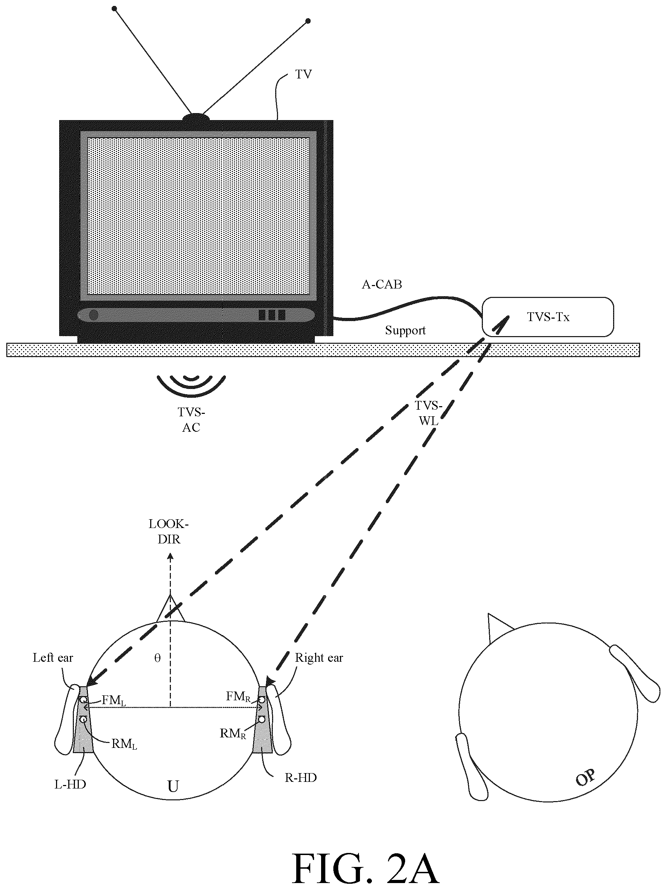

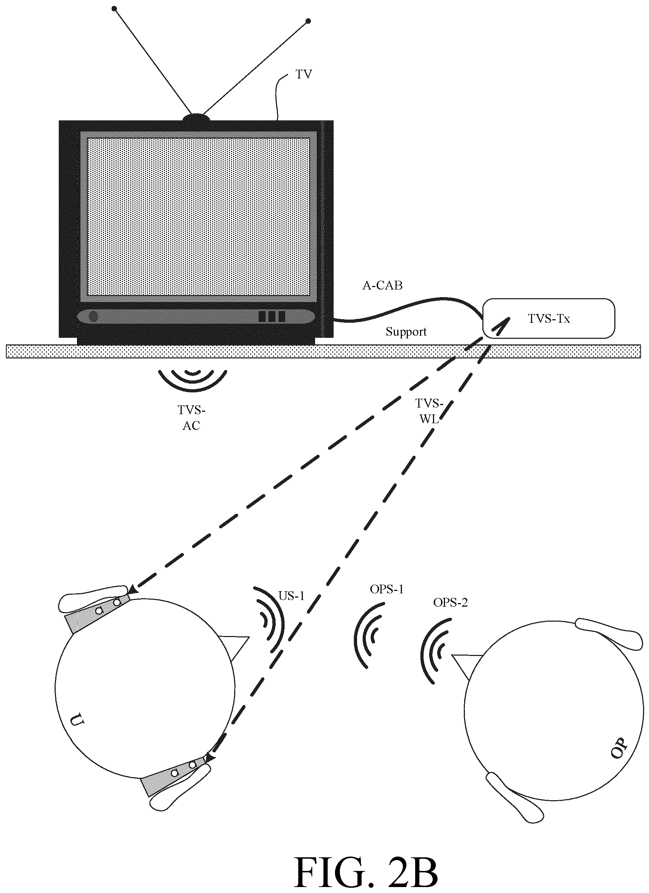

FIG. 2A shows a first TV-scenario comprising a hearing system according to the present disclosure; and FIG. 2B shows a second TV-scenario comprising a hearing system according to the present disclosure.

FIG. 2A and 2B illustrates an example of a situation where a hearing aid user (U) watches television (TV), or other apparatus providing images and accompanying sound, together with another person (OP), not necessarily wearing hearing aids. The hearing aid or hearing aid system (e.g. binaural hearing aid system) may be in a 'TV-reception mode' (where the hearing aid is configured to receive audio from an audio transmitter of a TV) in a one-way transmission from the TV (or TV-sound transmitter, or similar) to the hearing aid(s) (e.g. based on a preceding authentication procedure, e.g. a pairing procedure, between the transmitter and the hearing aid(s)). The TV-reception mode may be entered automatically or initiated by the user, e.g. by changing to a specific TV-reception mode, e.g. via a user interface. The accompanying sound may e.g. be provided acoustically (TVS-AC) via one or more built in (integrated) loudspeakers and/or one or more separate loudspeakers. The TV comprises or is connected to a transmitter (TVS-Tx) configured to transmit (e.g. wirelessly transmit) the sound (TVS-WL) from the TV to the hearing aid or (left and right) hearing aids (L-HD, R-HD) of the user (U). The left and right hearing aids are configured to be located at left and right ears, respectively, of the user. In the exemplary embodiment of FIG. 2A and 2B, the (or each of the) hearing aid(s) comprises two microphones, respective front (FM_{L}, FM_{R}) and rear (RM_{L}, RM_{R}) microphones located in respective front and rear parts of a BTE-part of the hearing aid (HD_{L}, HD_{R}). The hearing aid receives the TV sound acoustically (via the front and rear microphones (FM, RM) of FIG. 2A, 2B) (cf. also the input unit (IU_{MIC}) in FIG. 1). The hearing aid receives the TV sound wirelessly as well via appropriate (antenna and) wireless receiver circuitry, cf. bold dashed arrows (denoted TVS-WL) from the TV-sound transmitter (TVS-Tx) to each of the left and right hearing aids (HD_{L}, HD_{R}) (cf. also wireless receiver unit (IU_{AUX}) in FIG. 1). The wirelessly received TV sound (TVS-WL) is typically of a better quality (e.g. has a higher signal-to-noise ratio, the TV-sound being the (target) 'signal') than the acoustically propagated TV-sound (TVS-AC) and is hence, from a sound quality perspective, more attractive for the hearing aid user to listen to (it offers e.g. a better speech intelligibility). A down-side of entirely focusing on the wirelessly received TV sound is that sounds in the environment are not (or poorly) captured by the hearing aid user. The present disclosure offers a solution to this problem, as described in the following.

FIG. 2A shows a situation, where the user (U) and the other person (OP) watch the television (TV) in silence. In this situation (and in the absence of other persons), the hearing aid is configured to provide the user (U) with the wirelessly received sound (TVS-WL) from the transmitter (TVS-Tx). To give a little impression of the surrounding acoustic environment, the environment sound picked up by the front and rear microphones of the hearing aids is also presented to the hearing aid user via the respective output transducers (cf. OT of FIG. 1) of the left and right hearing aids (L-HD, R-HD) together with the wirelessly received sound, with a predefined (or adaptively defined) mixing ratio. The sound from the surrounding acoustic environment may be attenuated by a predefined (or dynamically determined) amount, e.g. between 10 and 30 dB, e.g. around 20 dB, compared to the wirelessly received sound (and/or compared to a normal presentation level of environment sound). Attenuation of the environment sound may constitute a default setting of the hearing aid in the TV-reception mode.

FIG. 2B shows a situation, where the user (U) and the other person (OP) talk together (cf. symbolic sound bites (time segments) (US-1, OPS-1, OPS-2)), while being less attentive to the 'output' of the TV. The symbolic sound bites are provided by the user (U: US-1) and the other person (OP: OPS-1, OPS2), respectively. In this situation, e.g. triggered by the detection of the user's voice, another weight to the acoustically propagated sound relative to the wirelessly received sound may be preferable for the user, e.g. if a conversation is initiated with the other person (OP), as indicated in FIG. 2B by the user (U) and the other person (OP) turning their heads towards each other. When the user starts to talk (as e.g. detected by an own voice detector) or when a conversation is identified (cf. e.g. FIG. 3A, 3B, 3C, 3D), attenuation of the sound from the surrounding acoustic environment may cancelled (or be reduced by a specific e.g. initial) amount) and then fully removed in dependence of the confidence in the conversation detection) thereby enabling a conversation between the hearing aid user and the other person to be appropriately conducted without disturbance of the TV-sound (for the hearing aid user).

When the conversation ends (or is estimated to have ended), the relative attenuation of environment sound may be removed with a certain delay (e.g. 10 seconds). The (e.g. default) attenuation may be gradually reintroduced over a certain time period (e.g. over some seconds, 'fading' from no (or low) attenuation to higher attenuation).

### Example of identification of a conversation:

FIG. 3A-3D shows voice detection signals for identifying a conversation between the user and another person, where
- FIG. 3A shows an exemplary output control signal VADC of a (general) voice activity detector;
- FIG. 3B shows an exemplary output control signal UVC of an own voice detector;
- FIG. 3C shows an exemplary control signal OPVC derived from the control signals VADC and UVC of FIG. 3A and 3B respectively; and
- FIG. 3D shows an exemplary conversation identifier based on the control signals of FIG. 3A, 3B and 3C.

FIG. 3D shows a time sequence of a received electric input signal from the environment (or of a signal originating therefrom, e.g. a beamformed signal) of a hearing aid worn by a user (U) reflecting a conversation of the user (U) with another person (OP) as detected by an own voice detector (OVD) and a (general) voice activity detector (VAD). FIG. 3A and 3B shows the output control signals (VADC, UVC) of respective voice activity detectors (VAD) and own voice activity detectors (OVD). FIG 3C shows the logic combination OPVC = VADC NOT(UVC) of the output control signals (VADC, UVC) of FIG. 3A and 3B providing an identification of time segments of speech from (any) other person than the user of the hearing aid (e.g. the 'other person' (OP) of FIG. 2A, 2B).

FIG. 3A, 3B, 3C shows values of different voice indicators (here control signals VADC representing any voice), UVC (representing the user's voice) and OPVC (representing other voice(s) than the user's)) versus time (Time) for a time segment of an electric input signal of the hearing aid (or a signal originating therefrom). FIG. 3D shows an output of a voice activity detector that is capable of differentiating a user's voice from other voices in an environment of the user wearing the hearing aid. The vocal activity or inactivity of the user or other persons is implied by control signals UVC or OPVC, respectively, being 1 or 0 (could also or alternatively be indicated by a speech presence probability (SPP) being above or below a threshold, respectively). In the time sequence depicted in FIG. 3C, the graph represents vocal activity of the other person (OP, speaking in time sequences OPS-1, OPS-2 in FIG. 2B) (between time t_{o,1} and t_{o,2} (time period Δt(OPS-1) = t_{o,2} - t_{o,1}) and between time t_{o,3} and t_{o,4} (time period Δt(OPS-2) = t_{o,4} - t_{o,3}). FIG. 3B represents vocal activity of the user (between time t_{u,1} and t_{u,2} (time period Δt(US-1) = t_{o,2} - t_{0,1}) and the graph in FIG. 3D represents vocal activity of the user and the other person(s) in combination. In FIG. 3D, time periods of the user' voice (denoted US-1) and other persons' voice (denoted OPS-1, OPS-2) are indicated by different filling. An analysis of the combination of indicators (UVC and OVC, respectively) of the presence or absence of user voice and other persons' voice may reveal a possible conversation with participation of the user. Identification of conversation involving the user may be identified by a sequential (alternating) occurrence of user voice (UVC) and other voice (OVC) indicators over a time period. In the simplified example of FIG. 3, a conversation involving the user from time t_{o,1} to t_{o,4} (i.e. over a total time period of t_{o,4} - t_{o,1} can be identified. During analysis, a criterion regarding the distance in time between the user voice indicator (UVC) shifting from active to inactive and the other person's voice indicator (OPVC) shifting from inactive to active (or vice versa) may be applied. Such criterion may e.g. be Δt(OPS-1->US-1) = t_{u,1}-t_{o,2} ≤ 2 s. A slight overlap of the two time segments (control signals) may be accepted, and a further criterion may e.g. be Δt(OPS-1->US-1) = t_{u,1}-t_{o,2} ≥ -2 s (thereby accepting a small period of 'double-talk').

FIG. 4A shows a time sequence of voice detection control signals reflecting a varying acoustic environment of the user of the hearing aid, including sub-sequences reflecting a varying degree of speech-participation by the user. FIG. 4A schematically illustrates a time window comprising time dependent values of indictors of the user's voice (UVC) and other person's voice (OPVC). The time window comprises a first time period that indicate a user in conversation with another person, a second time period of another persons' voice (without user participation, e.g. reflecting another person talking (without the user replying), e.g. voice from a radio, TV or other audio delivery device, or a person talking in the environment of the user), and a third time period where the user is talking alone, e.g. because he or she is in a telephone conversation (or is talking for a longer time (e.g. > 30 s) to another person in the room). Two time periods, that indicate silence (or no significant voice activity) separate the first second and third time periods. The time window of FIG. 4A has a range from t₁ to t₆, i.e. spans a time period of duration Δt_{w} from t₆ - t₁. The time window of FIG. 4A comprises in consecutive order: a period of 'conversation', a 1^{st} period of 'silence', a period of 'one way speech' (by another person than the user), a 2^{nd} period of 'silence', and a period of 'one way speech' (by the user).

FIG. 4B schematically shows an exemplary microphone gain modification versus time for a hearing aid according to the present disclosure when receiving streamed audio from first types of audio transmitters. FIG. 4B shows respective graphs indicating enablement ('Active') of reception of streamed audio from different (first) types of audio transmitters and corresponding gain modification (G_{ENV}) versus time when exposed to the different 'acoustic environments' of FIG. 4A. When transmission from these first types of transmitters is enabled (active=1), the gain modification (G_{ENV}) of the environment signal picked up by the hearing aid microphone(s) is constantly attenuated compared to a normal mode of operation (without receiving streamed audio), G_{ENV} = -A1, e.g. -20 dB.

The first types of transmitters are symbolically indicated in the left side of FIG. 4B and comprise:
- External microphone worn by a user to pick up own voice during a telephone conversation,
- Cellular telephones (Android- or IOS-based) when used for a telephone conversation (two-way audio), and
- Cellular telephones when used for one way audio (e.g. music) in a 'personal mode' where attention to surrounding sounds of the environment is minimum.

FIG. 4C schematically shows an exemplary microphone gain modification versus time for a hearing aid according to the present disclosure when receiving streamed audio from second types of audio transmitters. As FIG. 4B, FIG. 4C shows respective graphs indicating enablement ('Active') of reception of streamed audio from different (second) types of audio transmitters and corresponding gain modification (G_{ENV}) versus time when exposed to the different 'acoustic environments' of FIG. 4A. When transmission from these second types of transmitters is enabled (active=1), the gain modification (G_{ENV}) of the environment signal picked up by the hearing aid microphone(s) is attenuated when the user's voice (or a conversation) is detected compared to a normal mode of operation (without receiving streamed audio), G_{ENV} = -A2, e.g. -10 dB. The attenuation values A1 and A2 may be equal or different (e.g. user configurable, e.g. via a user interface).

The second types of transmitters are symbolically indicated in the left side of FIG. 4B and comprise:
- External microphone worn by another person than the user to pick the voice of the other person for transmission to the hearing of the hearing aid user,
- TV set (or other audio/video device) where one-way audio accompanying video images is transmitted to the hearing aid(s) of the user, and
- Cellular telephones (Android- or IOS-based) when used one way audio (e.g. music) in an 'environment mode', where attention to surrounding sounds of the environment is prioritized.

The attenuation values (A = A1 = A2, or A1 ≠ A2) may be adaptively determined in dependence of a current input level (e.g. a larger attenuation the larger the current input level, e.g. adaptively adjusted to the input level over time during the audio reception).

### Different scenarios:

The input gain controller is configured to apply an input gain to the electric input signal, or to a signal originating therefrom in dependence of a) the own voice control signal and b) the type of audio transmitter.

Some exemplary scenarios are given in the following.

The type of transmitter may e.g. be indicated by an audio transmitter type control signal provided by the wireless receiver or provided by the user via a user interface or extracted from the transmission format of the received wireless signal (e.g. defined by a transmission protocol of the transmitter from which the wireless signal is currently received).

The type of audio transmitter may e.g. be indicated by a current mode of operation of the hearing aid, e.g. defined by a current hearing aid program (or combination of hearing aid settings). The mode of operation may be automatically determined, e.g. by the wireless receiver, e.g. derived from the currently received wireless signal (e.g. from the protocol). The mode of operation may be manually determined, e.g. via a user interface of the hearing aid.

The hearing aid may be configured to operate in a multitude of different modes, e.g. a normal mode, and one or more specific modes, e.g. selectable by a user via a user interface, or automatically selectable. A mode of operation may be optimized to a specific acoustic situation or environment, e.g. a communication mode, such as a telephone mode (where the hearing aid is configured to receive audio from an audio transmitter of a telephone device and to transmit audio (the hearing aid user's voice) to a telephone device), or a partner microphone mode (wherein the hearing aid is configured to receive audio from an audio transmitter of a portable microphone), or a table microphone mode (where the hearing aid is configured to receive audio from an audio transmitter of a stationary (e.g. table-) microphone unit), or a TV-reception mode (where the hearing aid is configured to receive audio from an audio transmitter of a TV), etc.

### TV-sound (or similar) reception (e.g. in a 'TV-reception mode', cf. FIG. 4C):

- One way audio (from TV to hearing aid(s)).
- Sound from the environment (picked up by one or more microphones of the hearing aid) should be amplified (less attenuated, e.g. G_{ENV}=0 dB) when presented to the hearing aid user, if own-voice is detected, and not amplified (attenuated, e.g. G_{ENV}=-A dB), if no own voice is detected.

### External microphone (EM) sound (other person's voice) (EM-Other person (partner or table) microphone mode (cf. FIG. 4C, similar to TV-reception mode):

In situations when sound from another person than the hearing aid user is picked up by the external microphone (e.g. using a first (e.g. proprietary) transmission protocol):
- One way audio (from external microphone to hearing aid(s)).
- Sound from the environment (picked up by one or more microphones of the hearing aid) should be amplified (less attenuated, e.g. G_{ENV}=0 dB) when presented to the hearing aid user, if own-voice is detected (to be able to hear voices in the environment, not arriving from the external microphone), and not amplified (but attenuated, e.g. G_{ENV}=-A dB), if no own voice is detected.

### External microphone (EM) sound (hearing aid user's voice) (EM-Own voice mode)(cf. FIG. 4B, similar to telephone communication mode):

In situations when sound from the hearing aid user is picked up by the external microphone (e.g. using a second (e.g. standardized) transmission protocol (e.g. BLE)):
- Two way audio (between external microphone and hearing aid(s), e.g. part of a telephone conversation). The user will concentrate on the telephone conversation.
- Sound from the environment (picked up by one or more microphones of the hearing aid) should NOT be amplified (but attenuated) when presented to the hearing aid user, if own-voice is detected.

### Telephone sound (two-way) (e.g. in a 'Telephone communication-mode', cf. FIG. 4B):

In situations where an audio stream is received from a telephone and the user's own voice is picked up by the microphones of the hearing aid and transmitted to the telephone (e.g. via a standardized protocol (e.g. LEA2 (iOS based telephones) or ASHIA (Android-based telephones):
- Two way audio (between telephone and hearing aid(s) forming part of a telephone conversation).
- Sound from the environment (picked up by one or more microphones of the hearing aid) should NOT be amplified when presented to the hearing aid user, if own-voice is detected (nor if own voice is NOT detected).

### Telephone sound (one-way) (e.g. in a 'Telephone audio streaming (PA or EA)-mode':

In situations where an audio stream is received from a telephone and the user's own voice is NOT picked up by the microphones of the hearing aid and transmitted to the telephone:
- One way audio (from telephone to hearing aid(s)).
- User configuration may decide between a 'Personal Audio' mode and an 'Open Audio' mode of operation, respectively:
   ∘ Personal audio (PA) mode: Sound from the environment (picked up by one or more microphones of the hearing aid) should NOT be amplified when presented to the hearing aid user, if own-voice is detected (ignoring conversation) (cf. FIG. 4B), or
   ∘ Environment audio (EA) mode: Sound from the environment (picked up by one or more microphones of the hearing aid) should be amplified (less attenuated) when presented to the hearing aid user, if own-voice is detected (enabling a conversation) (cf. FIG. 4C).

### An Own Voice Predictor:

A long standing problem in hearing aids (e.g. hearing instruments) is the detection and processing of a hearing aid wearer's own voice. The problems are manifold, it is difficult to detect the difference between the wearer's voice and other people's voices, the processing of the wearer's voice is preferably different from the other voices, and the disadvantages of closed moulds leading to occlusion is much worse for own voice.

An electrical sensor close to the ear and close to skin, e.g. electroencephalography (EEG) or electromyography (EMG), connected to the hearing instrument(s) may pick up signals from the facial nerve and hereby detect that the wearer is going to move the jar and lips to speak. Since the electrical signals in the facial nerve happen before the speech is actually occurring, this system allows the hearing instrument to predict own voice before it happens and adjust the hearing instrument parameters accordingly (e.g. to reduce gain applied to the microphone-based signal presented to the user).

In one embodiment this enables the hearing instrument to reduce the gain, as well as adjusting other parameters, at the beginning where the own voice starts. Moreover, when the own voice is about to end, the hearing instrument may be configured to increases the gain, as well as to adjust other parameters, when the speech ends, hereby being ready to amplify other weaker speech signals in the surroundings. The analysis of facial nerve signals hereby enables the hearing instrument to amplify other people's voice more than the wearer's own voice and to switch between own voice processing on and off much faster and more aligned with the timing of the actual change (cf. e.g. FIG. 3D).

In another embodiment, the facial nerve signal is used to detect signals from the facial nerve prior to speech, which enables the hearing instrument to also increase the size (vent size) of a ventilation channel (e.g. a tube) to prevent occlusion. When the analysis of the facial nerve signal predicts the end of the speech signal, the vent size may be diminished. The analysis of the facial nerve signal hereby enables the provision of a larger vent during own voice where the gain is reduced (as well as other parameters) and a smaller vent when the wearer is not speaking out, and therefore requires more gain that could (otherwise with the increased vent size) cause howling.

### An own voice detector:

When a hearing aid user is talking, the input to the hearing aid from the user's vocal organs (from a distance of 0.1 to 0.3 m) is much louder than speech in a typical conversation (often at a distance of 1-2 meters). Own voice drives the hearing aid into (level) compression due to the level of the voice. Once the hearing aid is into compression, the amplification of other person's voices will often be insufficient and thereby inaudible. The time constants implemented in the compression rationale are hence important. If the hearing aid takes too long to go into compression, own voice will be too loud for the user. If the compressor takes too long to revert to previous settings the amplification of other voices will be too low. In practise the user experiences own voice as masker of other voices. This is especially important in discussions with fast turn taking.

Furthermore a 'correct' amplification of own voice is essential in order for the user to produce a correct level whilst speaking especially in situations where a change in level of own voice is expected.

It has until now not been possible to make a robust detection of own voice. Such a robust detection would enable a shift between two amplification schemes, one for talking and one for listening.

To develop a robust detection of own voice, electrophysiology recorded from the ear canal (earEEG) may provide a novel approach. EarEEG can be used to monitor the continuous *brain activity* recorded as electroencephalography (EEG). New research has shown that it is possible to detect EEG activity as speech production is in preparation prior to vocalization, se e.g. US2021235203A1, US2014369537A1, or US2015018699A1. Thus, this provides a predictive feature to switch amplification scheme even before own speech onset. On the other hand, producing speech involves a lot of *muscle activity,* which may also be recorded by the earEEG electrodes in terms of electromyography (EMG). EMG is characterized by high frequency content at high amplitudes and is easily detected in the signal. Hence, this provides a feature to detect ongoing speech, and the end of this signal provides a flag to when the amplification scheme should shift back to the listening scheme.

FIG. 5 illustrates a typical listening -> speech situation.

FIG. 5 schematically shows EEG signals originating from brain activity and muscle activity, respectively, of a hearing aid user in a listening situation (other voice) and a speech situation (own voice), respectively.

FIG. 5 schematically illustrates a typical conversation for a hearing impaired person. 1) The person is listening to speech (lower graph), and the hearing aid is in the listening scheme while it continuously records the brain activity (upper graph). 2) During the conversation, the person wants to reply, and while there is still a speech input, the hearing aid earEEG electrodes detects an alteration of the brain activity reflecting the cognitive processes underlying speech planning. Hence, the hearing aid shifts to the speaking scheme even before the person has started speaking. 3) During speech, the earEEG electrodes still records electrophysiology from the ear canal, and due to muscle activity, the signal is now characterized by high-frequency high-amplitude signals. In this state, the hearing aid is still in the speaking scheme. 4) After the person has spoken, the earEEG registers an end of the muscle activity and a return of the low-frequency low-amplitude signals reflecting a typical listening situation, and hence shifts back to the listening scheme.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element, but an intervening element may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

### REFERENCES

- US2011137649A1 (Oticon) 09.06.2011
- EP3930346A1 (Oticon) 29.12.2021
- US2021235203A1 (Oticon) 29.07.2021
- US2014369537A1 (Oticon) 18.12.2014
- US2015018699A1 (Univ. California, Trinity College) 15.01.2015

## Claims

1. A hearing aid configured to be worn by a user, the hearing aid comprising:
• a microphone configured to provide an electric input signal representative of sound from an environment around the user;
• a wireless receiver unit configured to receive a wireless signal from a transmitter of another device or system and to provide an audio input signal based thereon, and to identify said audio input signal as originating from one of a multitude of different types of audio transmitters;
• an own voice detector configured to provide an own voice control signal indicative of whether or not or with what probability the user's own voice is present in said sound from the environment of the user;
• a mixer configured to provide a mixed signal comprising a mixture of said electric input signal, or a signal originating therefrom and said audio input signal, or a signal originating therefrom;
• an input gain controller configured to apply an input gain to said electric input signal, or to a signal originating therefrom, at least when said hearing aid is in a wireless reception mode, wherein said wireless receiver unit receives a signal from at least one of said multitude of different types of audio transmitters; and
• an output transducer for providing stimuli representative of said mixed signal or a signal originating therefrom, perceivable as sound to the user; the hearing aid being **characterized in that** the input gain controller is configured to apply an input gain to said electric input signal, or to a signal originating therefrom in dependence of a) said own voice control signal and b) said type of audio transmitter.

2. A hearing aid according to claim 1, wherein said wireless receiver unit is configured to provide an audio transmitter type control signal indicative of the origin of a currently received wireless signal.

3. A hearing aid according to claim 1, wherein the input gain controller is configured to determine said type of audio transmitter from a current mode of operation of the hearing aid.

4. A hearing aid according to claim 3, wherein the current mode of operation of the hearing aid is determined by the user via a user interface.

5. A hearing aid according to any of one of claims 1-4, wherein at least two of said multitude of different types of audio transmitters use different audio transmission formats.

6. A hearing aid according to any one of claims 1-5, wherein said multitude of different types of audio transmitters comprise one or more of: a video-sound-transmitter, a table microphone transmitter, a portable microphone transmitter, and a telephone transmitter.

7. A hearing aid according to claim 5 or 6 as dependent on claim 5, wherein an audio transmission format of the different audio transmission formats comprises a standardized or proprietary audio transmission format.

8. A hearing aid according to any one of claims 1-7, the hearing aid comprising an other-voice detector configured to provide an other-voice-control signal indicative of whether or not or with what probability another voice than the user's own voice is present in the sound from the environment of the user.

9. A hearing aid according to any one of claims 1-8, the hearing aid comprising a conversation detector identifying a conversation that the user is currently engaged in, and to provide a conversation control signal indicative thereof.

10. A hearing aid according to claim 9, wherein the input gain controller is configured to apply said input gain to said electric input signal, or to a signal originating therefrom in dependence of a) said own voice control signal, b) said type of audio transmitter, and c) said conversation control signal.

11. A hearing aid according to any one of claims 1-10, wherein said input gain controller is configured to apply an input gain to said audio input signal.

12. A hearing aid according to any one of claims 1-11 wherein said input gain controller is configured to apply an input gain to said electric input signal and/or to said audio input signal to provide a certain mixing ratio of the mixed signal.

13. A hearing aid according to any one of claims 1-12, the hearing aid comprising one or more electrical sensors configured to be located close to the ear and close to skin of the user when the hearing aid is worn by the user.

14. A hearing aid according to claim 13, the hearing aid being configured to extract electroencephalography, EEG, and/or electromyography, EMG, signals from said one of more electrical sensors.

15. A hearing aid according to claim 14, wherein said own voice detector is based on an analysis of said EEG and/or EMG signals.

16. A hearing aid according to claim 15, wherein said own voice control signal is determined from a high pass filtered part of said EEG and/or said EMG signal(s).

## Patentansprüche

1. Hörgerät, das dazu konfiguriert ist, durch einen Benutzer getragen zu werden, wobei das Hörgerät Folgendes umfasst:
• ein Mikrofon, das dazu konfiguriert ist, ein elektrisches Eingangssignal bereitzustellen, das Schall aus einer akustischen Umgebung um den Benutzer herum darstellt;
• eine Drahtlosempfängereinheit, die dazu konfiguriert ist, ein drahtloses Signal von einem Sender einer anderen Vorrichtung oder eines anderen Systems zu empfangen und ein Audioeingangssignal auf Grundlage davon bereitzustellen und dieses Audioeingangssignal als von einem der Vielzahl von unterschiedlichen Typen von Audiotransmittern ausgehend zu identifizieren;
• einen Eigenstimmendetektor, der dazu konfiguriert ist, ein Eigenstimmensteuersignal bereitzustellen, das angibt, ob oder ob nicht oder mit welcher Wahrscheinlichkeit die Eigenstimme des Benutzers in dem Schall aus der Umgebung des Benutzers vorhanden ist;
• einen Mischer, der dazu konfiguriert ist, ein gemischtes Signal, das eine Mischung des elektrischen Eingangssignals oder eines davon stammenden Signals und des Audioeingangssignals umfasst, oder ein davon stammendes Signal bereitzustellen;
• eine Eingangsverstärkungssteuerung, die dazu konfiguriert ist, eine Eingangsverstärkung auf das elektrische Eingangssignal oder ein davon stammendes Signal mindestens dann anzuwenden, wenn das Hörgerät in einem drahtlosen Empfangsmodus ist, wobei die Drahtlosempfängereinheit ein Signal von mindestens einem der Vielzahl von unterschiedlichen Typen von Audiotransmittern empfängt; und
• einen Ausgangswandler zum Bereitstellen von Reizen, die die gemischten Signale oder ein davon stammendes Signal darstellen, die für den Benutzer als Schall wahrnehmbar sind;
wobei das Hörgerät **dadurch gekennzeichnet ist, dass**
die Eingangsverstärkungssteuerung dazu konfiguriert ist, eine Eingangsverstärkung auf das elektrische Eingangssignal oder ein davon stammendes Signal in Abhängigkeit von a) dem Eigenstimmensteuersignal und b) des Typs von Audiotransmitter anzuwenden.

2. Hörgerät nach Anspruch 1, wobei die Drahtloseempfängereinheit dazu konfiguriert ist, ein Steuersignal vom Audiotransmittertyp bereitzustellen, das den Ursprung des gegenwärtig empfangenen drahtlosen Signals angibt.

3. Hörgerät nach Anspruch 1, wobei die Eingangsverstärkungssteuerung dazu konfiguriert ist, den Typ von Audiotransmitter aus einem aktuellen Betriebsmodus des Hörgeräts zu bestimmen.

4. Hörgerät nach Anspruch 3, wobei der aktuelle Betriebsmodus des Hörgeräts durch den Benutzer über eine Benutzerschnittstelle bestimmt wird.

5. Hörgerät nach einem der Ansprüche 1-4, wobei mindestens zwei der Vielzahl von unterschiedlichen Typen von Audiotransmittern unterschiedliche Audioübertragungsformate verwenden.

6. Hörgerät nach einem der Ansprüche 1-5, wobei die Vielzahl von unterschiedlichen Typen von Audiotransmittern eines oder mehrere von Folgendem umfasst: einen Video-Schall-Transmitter, einen Transmitter für ein Tischmikrofon, einen Transmitter für ein tragbares Mikrofon und einen Telefontransmitter.

7. Hörgerät nach Anspruch 5 oder 6, wenn abhängig von Anspruch 5, wobei ein Audioübertragungsformat der unterschiedlichen Audioübertragungsformate ein standardisiertes oder proprietäres Audioübertragungsformat umfasst.

8. Hörgerät nach einem der Ansprüche 1-7, wobei das Hörgerät einen Fremdstimmendetektor umfasst, der dazu konfiguriert ist, ein Fremdstimmensteuersignal bereitzustellen, das angibt, ob oder ob nicht oder mit welcher Wahrscheinlichkeit eine anderer Stimme als die Eigenstimme des Benutzers in dem Schall aus der Umgebung des Benutzers vorhanden ist.

9. Hörgerät nach einem der Ansprüche 1-8, wobei das Hörgerät einen Konversationsdetektor umfasst, der eine Konversation identifiziert, an der der Benutzer gegenwärtig beteiligt ist, und ein Konversationssteuersignal bereitstellt, das dies angibt.

10. Hörgerät nach Anspruch 9, wobei die Eingangsverstärkungssteuerung dazu konfiguriert ist, eine Eingangsverstärkung auf das elektrische Eingangssignal oder ein davon stammendes Signal in Abhängigkeit von a) dem Eigenstimmensteuersignal und b) dem Typ von Audiotransmitter und c) dem Konversationssteuersignal anzuwenden.

11. Hörgerät nach einem der Ansprüche 1-10, wobei die Eingangsverstärkungssteuerung dazu konfiguriert ist, eine Eingangsverstärkung auf das Audioeingangssignal anzuwenden.

12. Hörgerät nach einem der Ansprüche 1-11, wobei die Eingangsverstärkungssteuerung dazu konfiguriert ist, eine Eingangsverstärkung auf das elektrische Eingangssignal und/oder das Audioeingangssignal anzuwenden, um ein bestimmtes Mischverhältnis des gemischten Signals bereitzustellen.

13. Hörgerät nach einem der Ansprüche 1-12, wobei das Hörgerät einen oder mehrere elektrische Sensoren umfasst, die dazu konfiguriert sind, sich nahe an dem Ohr und nahe an der Haut des Benutzers zu befinden, wenn das Hörgerät durch den Benutzer getragen wird.

14. Hörgerät nach Anspruch 13, wobei das Hörgerät dazu konfiguriert ist, Elektroenzephalographiesignale (EEG-Signale) und/oder Elektromyographiesignale (EMG-Signale) von dem einen oder den mehreren elektrischen Sensoren zu extrahieren.

15. Hörgerät nach Anspruch 14, wobei der Eigenstimmendetektor auf einer Analyse der EEG- und/oder EMG-Signale basiert.

16. Hörgerät nach Anspruch 15, wobei das Eigenstimmensteuersignal von einem hochpassgefilterten Teil der EEG- und/oder der EMG-Signale bestimmt wird.

## Revendications

1. Prothèse auditive conçue pour être portée par un utilisateur, la prothèse auditive comprenant :
• un microphone conçu pour fournir un signal d'entrée électrique représentatif d'un son à partir d'un environnement autour de l'utilisateur ;
• une unité de réception sans fil conçue pour recevoir un signal sans fil à partir d'un transmetteur d'un autre dispositif ou système et pour fournir un signal d'entrée audio sur la base de celui-ci, et pour identifier ledit signal d'entrée audio comme provenant de l'un parmi une multitude de types différents de transmetteurs audio ;
• un détecteur de sa propre voix conçu pour fournir un signal de commande de sa propre voix indiquant si oui ou avec quelle probabilité la propre voix de l'utilisateur est présente dans ledit son à partir de l'environnement de l'utilisateur ;
• un mélangeur conçu pour fournir un signal mélangé comprenant un mélange dudit signal d'entrée électrique, ou d'un signal provenant de celui-ci et dudit signal d'entrée audio, ou d'un signal provenant de celui-ci ;
• un dispositif de commande de gain d'entrée conçu pour appliquer un gain d'entrée audit signal d'entrée électrique, ou à un signal provenant de celui-ci, au moins lorsque ladite prothèse auditive est dans un mode de réception sans fil, dans laquelle l'unité de réception sans fil reçoit un signal à partir d'au moins l'un parmi ladite multitude de types différents de transmetteurs audio ; et
• un transducteur de sortie destiné à fournir des stimuli représentatifs dudit signal mélangé ou d'un signal provenant de celui-ci, perceptibles sous forme de son pour l'utilisateur ;
la prothèse auditive étant **caractérisée en ce que** le dispositif de commande de gain d'entrée est conçu pour appliquer un gain d'entrée audit signal d'entrée électrique, ou à un signal provenant de celui-ci en fonction a) dudit signal de commande de sa propre voix et b) dudit type de transmetteur audio.

2. Prothèse auditive selon la revendication 1, dans laquelle l'unité de récepteur sans fil est conçue pour fournir un signal de commande de type transmetteur audio indiquant l'origine d'un signal sans fil actuellement reçu.

3. Prothèse auditive selon la revendication 1, dans laquelle le dispositif de commande de gain d'entrée est conçu pour déterminer ledit type de transmetteur audio à partir d'un mode de fonctionnement actuel de la prothèse auditive.

4. Prothèse auditive selon la revendication 3, dans laquelle le mode de fonctionnement actuel de la prothèse auditive est déterminé par l'utilisateur par l'intermédiaire d'une interface utilisateur.

5. Prothèse auditive selon l'une quelconque des revendications 1 à 4, dans laquelle au moins deux parmi ladite multitude de types différents de transmetteurs audio utilisent des formats de transmission audio différents.

6. Prothèse auditive selon l'une quelconque des revendications 1 5, dans laquelle ladite multitude de différents types de transmetteurs audio comprennent un ou plusieurs parmi : un transmetteur de son vidéo, un transmetteur de microphone de table, un transmetteur de microphone portable et un transmetteur de téléphone.

7. Prothèse auditive selon la revendication 5 ou 6 telle que dépendante de la revendication 5, dans laquelle un format de transmission audio parmi les différents formats de transmission audio comprend un format de transmission audio normalisé ou propriétaire.

8. Prothèse auditive selon l'une quelconque des revendications 1 à 7, la prothèse auditive comprenant un détecteur d'autre voix conçu pour fournir un signal de commande d'autre voix indiquant si oui ou avec quelle probabilité une autre voix que la propre voix de l'utilisateur est présente dans le son provenant de l'environnement de l'utilisateur.

9. Prothèse auditive selon l'une quelconque des revendications 1 à 8, la prothèse auditive comprenant un détecteur de conversation identifiant une conversation à laquelle l'utilisateur participe actuellement, et pour fournir un signal de commande de conversation indiquant celle-ci.

10. Prothèse auditive selon la revendication 9, dans laquelle le dispositif de commande de gain d'entrée est conçu pour appliquer ledit gain d'entrée audit signal d'entrée électrique, ou à un signal provenant de celui-ci en fonction a) dudit signal de commande de sa propre voix, b) dudit type de transmetteur audio, et c) dudit signal de commande de conversation.

11. Prothèse auditive selon l'une quelconque des revendications 1 à 10, dans laquelle ledit dispositif de commande de gain d'entrée est conçu pour appliquer un gain d'entrée audit signal d'entrée audio.

12. Prothèse auditive selon l'une quelconque des revendications 1 à 11, dans laquelle ledit dispositif de commande de gain d'entrée est conçu pour appliquer un gain d'entrée audit signal d'entrée électrique et/ou audit signal d'entrée audio pour fournir un certain rapport de mélange du signal mélangé.

13. Prothèse auditive selon l'une quelconque des revendications 1 à 12, la prothèse auditive comprenant un ou plusieurs capteurs électriques conçus pour être situés à proximité de l'oreille et à proximité de la peau de l'utilisateur lorsque la prothèse auditive est portée par l'utilisateur.

14. Prothèse auditive selon la revendication 13, la prothèse auditive étant conçue pour extraire des signaux d'électroencéphalographie EEG, et/ou d'électromyographie, EMG, à partir desdits un ou plusieurs capteurs électriques.

15. Prothèse auditive selon la revendication 14, dans laquelle ledit détecteur de sa propre voix est basé sur une analyse desdits signaux EEG et/ou EMG.

16. Prothèse auditive selon la revendication 15, dans laquelle ledit signal de commande de sa propre voix est déterminé à partir d'une partie filtrée passe-haut dudit EEG et/ou dudit ou desdits signaux EMG.
